# EUROPÄISCHE PATENTSCHRIFT

(11) **EP 1 841 386 B1**
(45) Veröffentlichungstag und Bekanntmachung des Hinweises auf die Patenterteilung: **18.05.2011**
(21) Anmeldenummer: 05787619.5
(22) Anmeldetag: 19.09.2005
(51) Int. Cl.: A61F 2/44

(54) **ZERVIKALE ZWISCHENWIRBELPROTHESEN**
CERVICAL INTERVERTEBRAL PROSTHESES
PROTHESES INTERVERTEBRALES CERVICALES

(30) Priorität: 26.01.2005 EP 05001557
(43) Veröffentlichungstag der Anmeldung: 10.10.2007
(73) Patentinhaber: Cervitech, Inc., San Diego CA 92121 (US)
(72) Erfinder: KELLER, Arnold, 23863 Kayhude (DE)
(74) Vertreter: Glawe, Delfs, Moll
(86) Internationale Anmeldenummer: PCT/EP2005/010081
(87) Internationale Veröffentlichungsnummer: WO 2006/079366

(56) Entgegenhaltungen:
- EP-A- 1 374 807
- US-A- 5 755 796
- US-A- 5 899 941
- US-B1- 6 368 350

## Beschreibung

Es ist eine zervikale Zwischenwirbelprothese bekannt (US 6368350), die aus einer oberen und einer unteren Anschlußplatte zum Anschluß an die benachbarten Wirbelkörper und einem Gelenkkern besteht, der mit der einen Anschlußplatte ein Gelenk bildet, das aus einer konvexen Gelenkfläche an der Unterseite der oberen Anschlußplatte und einer konkaven Gelenkfläche an der Oberseite des Gelenkkerns besteht. An der anderen Anschlußplatte stützt sich der Gelenkkern über eine Gleitfläche ab, die parallel zur Erstreckungsrichtung der Anschlußplatte verläuft und dem Gelenkkern in dieser Richtung eine translatorische Relativbewegung im Verhältnis zur unteren Anschlußplatte gestattet. Diese Relativbewegung wird dadurch begrenzt, daß die Gleitfläche von einem hochstehenden Rand begrenzt wird. Die Gelenkfläche des Gelenkkerns ist bei der bekannten Prothese sehr flach schalenförmig ausgebildet. Dies ist üblich, weil man dadurch eine geringe spezifische Flächenbelastung erreicht. Es bringt aber die Gefahr mit sich, daß dann, wenn die Bänder, die die beteiligten Wirbelkörper miteinander verbinden, zu schwach oder gedehnt sind, die Prothesenteile sich voneinander lösen. Zwar ist es bekannt (US-A-5895428), die Prothesenteile durch eine mittig durch die Gelenkflächen passierende Schraube miteinander zu verbinden; das läßt sich aber nicht mit der oben erwähnten translatorischen Bewegungsfreiheit des Gelenkkerns parallel zur Erstreckung der Anschlußplatten verbinden und ist im übrigen bei einer Zervikalprothese wegen der beengten Raumverhältnisse unmöglich.

Der Erfindung liegt daher die Aufgabe zugrunde, eine Prothese der eingangs genannten Art zu schaffen, die hinreichende Stabilität auch im Falle schwacher Bänder verspricht. Die Lösung besteht in den Merkmalen des Hauptanspruchs sowie vorzugsweise in den Merkmalen der Unteransprüche.

Demnach ist vorgesehen, daß die konkave Gelenkfläche die konvexe Gelenkfläche in einem Raumwinkel von mindestens 90° umfaßt Dadurch ist sichergestellt, daß schräg von der Seite her eintreffende Kräfte die konvexe Gelenkfläche nicht aus der konkaven Gelenkfläche herausschieben können. Weil der genannte Winkel den Bereich angibt, in welchem die konkave Gelenkfläche die konvexe Gelenkfläche umfaßt, wird er im folgenden als Umfassungswinkel bezeichnet. Zweckmäßigerweise beträgt er mindestens 110°, weiter vorzugsweise 135°. Wenn er nach einem weiteren Merkmal der Erfindung über 180° liegt, ist die konvexe Gelenkfläche gänzlich in der konkaven Gelenkfläche gesichert. Der Umfassungswinkel bzw. Raumwinkel wird als Zentriwinkel eines Kreiskegels zwischen Radien gemessen, die von gegenüberliegenden Punkten des Rands der konkaven Gelenkfläche ausgehen.

Da die Stabilisierung der Prothese im Bereich der Gelenkflächen vergeblich wäre, wenn die Verbindung des Gelenkkerns mit der anderen Anschlußplatte unstabil ist, sieht die Erfindung ferner vor, daß der in dem Raumwinkel von mindestens 90° gelegene Teil der konkaven Gelenkfläche in der von dem Rand eingeschlossenen Ausnehmung liegt. Das heißt, daß dieser Teil der Gelenkfläche unterhalb der von der Oberkante des Rands aufgespannten Fläche liegt. Dadurch wird bewirkt, daß der Gelenkkern durch seitlich eintreffende Kräfte mit hinreichender Wahrscheinlichkeit nicht aus der Ausnehmung der betreffenden Anschlußplatte herausgehoben werden kann. Wenn, wie oben als vorteilhaft geschildert, ein über 90° liegender Umfassungswinkel gewählt wird, so muß nicht auch der über 90° hinausgehende Teil der konkaven Gelenkfläche in der Ausnehmung liegen. Jedoch ist dies vorteilhaft.

Die Positionierung der konvexen Gelenkfläche innerhalb der Ausnehmung dient nicht nur der Sicherung des Gelenkkerns an der in haltenden Anschlußplatte sondern auch der Verringerung der Bauhöhe der Prothese. Der Radius der konvexen Gelenkfläche wird so klein im Verhältnis zum Querschnitt des Gelenkkerns bemessen, daß sie zum größeren Teil innerhalb desjenigen Teils des Gelenkkerns liegt, der von der Ausnehmung der unteren Anschlußplatte umschlossen ist. Der Gelenkkern kann so ausgebildet sein, daß er im wesentlichen ausschließlich innerhalb der Ausnehmung liegt. Er kann jedoch mit einem Kragen versehen sein, der oben auf dem Rand des Ausnehmung aufliegt, um bei Beugung des Gelenks einen unmittelbaren Kontakt der Metallteile der oberen und unteren Anschlußplatten auszuschließen.

Die Ausnehmung soll den Gelenkkern wenigstens bis zu einem Drittel, vorzugsweise bis etwa zur Hälfte seines Gelenkflächenradius aufnehmen. Besonders bevorzugt wird eine Ausführung, bei welcher die Ausnehmung den Gelenkkern im wesentlichen bis zur Höhe des Mittelpunkts seiner Gelenkfläche aufnimmt. Die Mindesthöhe des die Ausnehmung umgebenden Randes über der Gleitfläche soll mindestens ein Viertel, vorzugsweise mindestens ein Drittel der AP-Ausdehnung der Ausnehmung betragen. Die Abkürzung AP bedeutet die anteroposteriore Richtung.

Durch das Zusammenwirken des Gelenkkerns mit dem Rand der Ausnehmung kann die Bewegungsfreiheit des Gelenkkerns in gewünschter Weise vorherbestimmt werden. Bei einer vorteilhaften Ausführungsform ist die Ausnehmung rechteckig und vorzugsweise quadratisch begrenzt und verlaufen ihre Begrenzungen parallel zur AP-Richtung und quer dazu. Dies hat den Vorteil, daß eine Ausnehmung von größtmöglicher Ausdehnung in der unteren Anschlußplatte untergebracht werden kann. Bei einer ersten Variante dieser Ausführungsform ist ein rechteckig begrenzter Gelenkkern verfügbar, der in einer Richtung eine kürzere Seitenlänge als die Ausnehmung aufweist, während seine andere Seitenlänge mit der Seitenlänge der Ausnehmung im wesentlichen übereinstimmt. Dadurch wird erreicht, daß je nach der Orientierung des Gelenkkerns in der Ausnehmung die Bewegungsfreiheit des Gelenkkerns auf die AP-Richtung (dies ist der häufigste Fall) oder die lotrecht dazu stehende Richtung beschränkt werden kann. Wenn in einer weiteren Variante die translatorische Bewegung des Gelenkkerns gänzlich ausgeschlossen werden soll, verwendet man einen Gelenkkern, der quadratisch mit denselben Abmessungen wie die Ausnehmung ist. Wenn hingegen Bewegungsfreiheit in allen Richtungen erwünscht ist, werden die Abmessungen des Gelenkkerns in allen Richtungen kleiner als die der Ausnehmung gewählt.

Die Erfindung wird im folgenden anhand der Zeichnung näher erläutert. Darin zeigen:
- Fig. 1: einen Sagittalschnitt durch eine erste und
- Fig. 2: einen Sagittalschnitt durch eine zweite Ausfüh- rungsform sowie
- Fig. 3: die auseinandergezogene perspektivische Darstellung zweier Anschlußplatten mit dazwischen dargestellten unterschiedlichen Versionen von Gelenkkernen.

Eine erste Anschlußplatte 1 weist eine Fläche 2 auf, die zur Verbindung mit der Deckplatte eines ersten Wirbelkörpers bestimmt ist. Sie kann mit Zähnen oder anderen Einrichtungen für die innige Verbindung mit dem Knochengewebe ausgerüstet sein. Ein ventraler Flansch 3 enthält eine Schraubenbohrung 4 für eine Knochenschraube. Es versteht sich, daß diese konstruktiven Einzelheiten der Prothese unterschiedlich ausgeführt werden können. Eine zweite Deckplatte 5 weist eine Oberfläche 6 zur Verbindung mit dem zweiten Wirbelkörper sowie einen ventralen Flansch 7 mit Schraubenbohrung 8 auf. Die erste Anschlußplatte 1 ist oben dargestellt und wird auch meistens oben verwendet. Im folgenden wird sie deshalb als obere Anschlußplatte und die andere als untere Anschlußplatte bezeichnet. Jedoch kann die Anordnung auch umgekehrt sein.

Die Unterseite der oberen Anschlußplatte trägt einen Gelenkkopf 10, der eine konvexe Gelenkfläche 11 bildet mit Mittelpunkt 12. Die untere Anschlußplatte 5 trägt einen umlaufenden Rand 15, der eine quadratische Ausnehmung 16 oberhalb einer ebenen Gleitfläche 17 umschließt. Die Innenflächen des Rands 15 verlaufen lotrecht zu der den Boden der Ausnehmung 16 bildenden Gleitfläche 17 sowie parallel zur AP-Richtung bzw. lotrecht dazu. Die Ecken 18 sind abgerundet. Die Abmessungen 26, 27 in AP-Richtung bzw. quer dazu sind gleich. Die Anschlußplatten 1, 5 bestehen zweckmäßigerweise aus widerstandsfähigem Material wie Metall.

Innerhalb der Ausnehmung 16 befindet sich der Gelenkkern 20, der zweckmäßigerweise aus Polyethylen, Keramik oder anderem gleitgünstigem Material besteht. Er weist eine ebene Unterfläche auf, die sich möglichst großflächig auf der Gleitfläche 17 abstützt und zusammen mit dieser ein Schiebelager für translatorische Bewegung bildet. Seine in AP-Richtung verlaufende Seitenfläche 21 und die dazu quer verlaufende Seitenfläche 22 bilden eine rechteckige Begrenzung, die der Begrenzung der Ausnehmung 16 durch die Innenflächen des Rands 15 komplementär ähnlich ist.

Der Gelenkkern 20 trägt am oberen Rand eine Kragen 25, der sich auf die Oberkante des Rands 15 auflegt. Im Falle einer Beugebewegung schützt er die Unterseite der oberen Anschlußplatte 1 vor einem direkten Anschlag an Teilen der unteren Anschlußplatte 5. In den seitlichen Bereichen 24 zwischen den Anschlußplatten 1, 5 verbleibt hinreichender Platz für eine relative Beugebewegung.

Der Gelenkkern 20 enthält eine sphärische Vertiefung, die komplementär der konvexen Gelenkfläche 11 gleicht und die zugehörige konkave Gelenkfläche bildet. Ihr Mittelpunkt 12 fällt mit demjenigen der konvexen Gelenkfläche 11 zusammen. Sie umfaßt die konvexe Gelenkfläche 11 in einem Umfassungswinkel 28 von mehr als 180 Grad. Oberhalb des Mittelpunkts 12 verengt sie sich ein wenig, so daß der Gelenkkopf 10 ihr nur unter elastischer Verformung entweichen kann und daher unter normalen Kräfteverhältnissen in ihr festgehalten wird.

infolge der weitgehenden Umfassung des Gelenkkopfes 10 durch die konkave Gelenkfläche 11 des Gelenkkerns 20 weist das Beugegelenk eine hohe Stabilität auch bei seitlich eintreffenden Kräften auf. Ebenso ist der Gelenkkern 20 mit hoher Stabilität innerhalb der Ausnehmung 16 von dem Rand 15 gehalten. Dies liegt daran, daß der Gelenkkern 20 nahezu vollständig, jedenfalls bis zu beträchtlicher Höhe, von dem Rand 15 umschlossen ist. Seine Oberkante und die von dieser aufgespannte Fläche 23 liegen höher als der Mittelpunkt 12 der konkaven Gelenkfläche 11. Die Höhe 29 des Randes 15 über der Gleitfläche 17 ist etwas größer als zwei Fünftel der AP-Abmessung 26 der Ausnehmung 16. Selbst wenn der Gelenkkern durch unvorhergesehene Kräfte ein wenig angehoben werden sollte, wird er durch den Rand 15 stets in die dargestellte Stellung zurückgeführt.

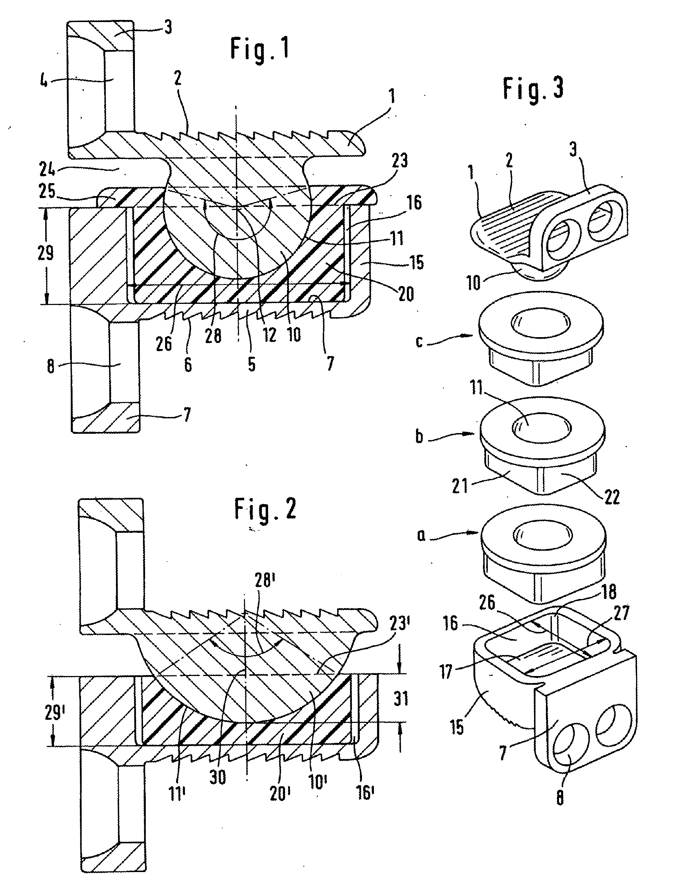

Die Anschlußplatten 1,5 können mit Gelenkkernen von unterschiedlichen Seitenlängen kombiniert werden. Dies ist in Figur 3 veranschaulicht. Die Abmessungen der Seitenflächen des Gelenkkerns "a" gleichen (mit einem angemessenen Spiel) den Innenflächen des Rands 15. Wenn er in die Ausnehmung 16 der unteren Anschlußplatte eingesetzt ist, besitzt er keine translatorische Relativbeweglichkeit im Verhältnis zu der unteren Anschlußplatte 5. Man verwendet ihn deshalb bei stark geschwächten Bändern, wenn die Prothese die Stabilisierung des Zwischenwirbelgelenks übernehmen muß.

Der Gelenkkern "c" ist in der AP-Richtung und quer dazu kleiner als die Ausnehmung 16. Folglich besteht eine begrenzte Bewegungsmöglichkeit in allen Richtungen. Man verwendet diesen Gelenkkern in denjenigen Fällen, in denen dem Gelenk eine gewisse translatorische Bewegungsstrecke zugemutet werden kann, aber nach dieser Bewegungsstrecke eine Stabilisierung erwünscht ist.

Der Gelenkkern "b" repräsentiert ein Mittelding zwischen den vorgenannten Möglichkeiten, da die Seitenlänge seiner Seitenflächen 21 den Seitenlängen 26, 27 der Ausnehmung 16 gleicht, während das quer dazu verlaufende Paar von Seitenflächen 22 kürzer ist. Das bedeutet, daß der Gelenkkern b gegenüber der zugehörigen unteren Anschlußplatte 5 in Richtung der Seitenflächen 21 unbeweglich, in Richtung der Seitenflächen 22 jedoch begrenzt beweglich ist. Diesen Gelenkkern setzt man dann ein, wenn die Notwendigkeit der Stabilisierung in unterschiedlichen Richtungen verschieden ist. Meist wird die Beweglichkeit in der AP-Richtung vorgesehen, während die Verbindung in der Querrichtung fest ist. Jeldoch kann man auch die umgekehrte Möglichkeit realisieren.

Figur 2 veranschaulicht ein Ausführungsbeispiel, daß sich von demjenigen der Figur 1 durch eine flachere Ausbildung der Gelenkflächen 11' und einen niedrigeren Gelenkkern 20' unterscheidet. Der Umfassungswinkel 28' beträgt nur etwa 110°. Der von ihm definierte Bereich der Gelenkfläche 11' liegt gänzlich innerhalb der Ausnehmung 16', d.h. nicht höher als die obere Begrenzungsfläche 23'. Der Radius 30 der Gelenkfläche liegt mit seinem Abschnitt 31 zu mehr als einem Drittel unterhalb der Begrenzungsfläche 23' innerhalb der Ausnehmung 16'. Die Höhe 29' der Ausnehmung 16' gleicht etwa einem Drittel der AP-Abmessung der Ausnehmung. Die Gesamthöhe der Prothese ist ein wenig geringer als die des ersten Ausführungsbeispiels. Gleichwohl liefert sie hohe Seitenstabilität.

## Patentansprüche

1. Zervikale Zwischenwirbelprothese mit zwei Anschlußplatten ((1, 5) zum Anschluß an je einen der benachbarten Wirbelkörper und mit einem Gelenkkern (20, 20'), der mit der einen Anschlußplatte (1) ein Gelenk mit einer konkaven Gelenkfläche (11, 11') an dem Gelenkkern (20, 20') und einer komplementär konvexen Gelenkfläche an der Anschlußplatte (1) bildet und sich an der anderen Anschlußplatte (5) über eine im wesentlichen parallel zur Richtung der Anschlußplatte (5) sich erstreckende Gleitfläche (17) abstützt, die eine Relativbewegung in dieser Richtung gestattet und von einem diese Relativbewegung begrenzenden Rand (15) umgeben ist, **dadurch gekennzeichnet, daß** die konkave Gelenkfläche (11, 11') die konvexe Gelenkfläche in einem Raumwinkel (28, 28') von mindestens 90° umfaßt und ihr in diesem Raumwinkel liegender Teil innerhalb der von dem Rand (15) umschlossenen Ausnehmung (16, 16') liegt.

2. Zwischenwirbelprothese nach Anspruch 1, **dadurch gekennzeichnet, daß** die konkave Gelenkfläche (11,) die konvexe Gelenkfläche in einem Raumwinkel (28) von mindestens 180° umfaßt.

3. Zwischenwirbelprothese nach Anspruch 1 oder 2, **dadurch gekennzeichnet, daß** die Ausnehmung (16, 16') den Gelenkkern (20, 20') wenigstens bis zur Höhe eines Drittels des Gelenkflächenradius (30) aufnimmt.

4. Zwischenwirbelprothese nach Anspruch 3, **dadurch gekennzeichnet, daß** die Ausnehmung (16) den Gelenkkern (20) im wesentlichen bis zur Höhe des Mittelpunkts (12) seiner Gelenkfläche (11) aufnimmt.

5. Zwischenwirbelprothese nach einem der Ansprüche 1 bis 4, **dadurch gekennzeichnet, daß** die Höhe (29) des die Ausnehmung (15) umgebenden Rands mindestens ein Viertel ihrer Erstreckung (26) in AP-Richtung beträgt.

6. Zwischenwirbelprothese nach einem der Ansprüche 1 bis 5, **dadurch gekennzeichnet, daß** der Gelenkkern (20) im wesentlichen vollständig innerhalb der Ausnehmung (16) liegt.

7. Zwischenwirbelprothese nach Anspruch 6, **dadurch gekennzeichnet, daß** der Gelenkkern (20) einen den unmittelbaren Kontakt der Anschlußplatten (1, 5) bei Beugung hindernden Kragen (25) trägt.

8. Zwischenwirbelprothese nach einem der Ansprüche 1 bis 7, **dadurch gekennzeichnet, daß** die Ausnehmung (16) rechteckig ist und ihre Seiten parallel bzw. lotrecht zur AP-Richtung verlaufen.

9. Zwischenwirbelprothese nach Anspruch 8, **dadurch gekennzeichnet, daß** die Ausnehmung (16) quadratisch ist und ein rechteckig begrenzter Gelenkkern (b) verfügbar ist, dessen eines Seitenpaar (21) eine mit der Seitenlänge (26, 27) der Ausnehmung (16) im wesentlichen übereinstimmende und dessen anderes Seitenpaar (22) eine kürzere Seitenlänge aufweist.

10. Zwischenwirbelprothese nach Anspruch 8, **dadurch gekennzeichnet, daß** ein Gelenkkern (a) verfügbar ist, der im wesentlichen dieselben Abmessungen wie die Ausnehmung (16) hat.

11. Zwischenwirbelprothese nach Anspruch 8, **dadurch gekennzeichnet, daß** die Ausnehmung (16) quadratisch begrenzt ist und ein rechteckig begrenzter Gelenkkern (b) verfügbar ist, der in beiden Richtungen eine geringere Seitenlänge als die Ausnehmung (16) hat.

## Claims

1. Cervical intervertebral prosthesis with two connection plates (1, 5) for connection to in each case one of the adjacent vertebral bodies, and with a hinge core (20, 20') which forms, together with one connection plate (1), a hinge with a concave hinge surface (11, 11') on the hinge core (20, 20') and with a complementary convex hinge surface on the connection plate (1), and which bears on the other connection plate (5) via a slide surface (17) which extends substantially parallel to the direction of the connection plate (5) and which permits a relative movement in this direction and is surrounded by an edge (15) limiting this relative movement, **characterized in that** the concave hinge surface (11, 11') encloses the convex hinge surface within a solid angle (28, 28') of at least 90°, and its part lying within this solid angle lies inside the recess (16, 16') surrounded by the edge (15).

2. Intervertebral prosthesis according to Claim 1, **characterized in that** the concave hinge surface (11) encloses the convex hinge surface within a solid angle (28) or at least 180°.

3. Intervertebral prosthesis according to Claim 1 or Claim 2, **characterized in that** the recess (16, 16') accommodates the hinge core (20, 20') at least to the height of one third of the hinge surface radius (30).

4. Intervertebral prosthesis according to Claim 3, **characterized in that** the recess (16) accommodates the hinge core (20) substantially to the height of the center point (12) of its hinge surface (11).

5. Intervertebral prosthesis according to one of Claims 1 to 4, **characterized in that** the height (29) of the edge surrounding the recess (15) is at least one quarter of its extent (26) in the AP direction.

6. Intervertebral prosthesis according to one of Claims 1 to 5, **characterized in that** the hinge core (20) lies substantially completely inside the recess (16).

7. Intervertebral prosthesis according to Claim 6, **characterized in that** the hinge core (20) has a collar (25) avoiding direct contact of the connection plates (1, 5) upon flexion.

8. Intervertebral prosthesis according to one of Claims 1 to 7, **characterized in that** the recess (16) is rectangular, and its sides run parallel and perpendicular to the AP direction.

9. Intervertebral prosthesis according to Claim 8, **characterized in that** the recess (16) is square, and a rectangularly delimited hinge core (b) is available whose one pair of sides (21) has a side length substantially corresponding to the side length (26, 27) of the recess (16) and whose other pair of sides (22) has a shorter side length.

10. Intervertebral prosthesis according to Claim 8, **characterized in that** a hinge core (a) is available which has substantially the same dimensions as the recess (16).

11. Intervertebral prosthesis according to Claim 8, **characterized in that** the recess (16) is square, and a rectangularly delimited hinge core (b) is available which, in both directions, has a shorter side length than the recess (16).

## Revendications

1. Prothèse intervertébrale cervicale dotée de deux plaques de raccordement (1, 5) qui sont destinées à être raccordées chacune à l'un des corps de vertèbre voisins et d'une âme d'articulation (20, 20') qui forme avec une plaque de raccordement (1) une articulation dotée d'une surface concave d'articulation (11, 11') sur l'âme d'articulation (20, 20') et d'une surface complémentaire convexe d'articulation sur la plaque de raccordement (1), et qui s'appuie sur l'autre plaque de raccordement (5) par une surface de coulissement (17) qui s'étend essentiellement en parallèle à la direction de la plaque de raccordement (5), qui permet un déplacement relatif dans cette direction et qui est entourée par une bordure (15) qui limite ses déplacements relatifs,
**caractérisée en ce que**
la surface concave d'articulation (11, 11') entoure la surface convexe d'articulation dans une plage angulaire (28, 28') d'au moins 90° et **en ce que**
sa partie située dans cette plage angulaire est située à l'intérieur de la découpe (16, 16') entourée par la bordure (15).

2. Prothèse intervertébrale selon la revendication 1, **caractérisée en ce que** la surface concave d'articulation (11) entoure la surface convexe d'articulation dans une plage angulaire (28) d'au moins 180°.

3. Prothèse intervertébrale selon les revendications 1 ou 2, **caractérisée en ce que** la découpe (16, 16') reprend l'âme d'articulation (20, 20') au moins jusqu'à la hauteur d'un tiers du rayon (30) de la surface d'articulation.

4. Prothèse intervertébrale selon la revendication 3, **caractérisée en ce que** la découpe (16) reprend l'âme d'articulation (20) essentiellement jusqu'à la hauteur du point central (12) de sa surface d'articulation (11).

5. Prothèse intervertébrale selon l'une des revendications 1 à 4, **caractérisée en ce que** la hauteur (29) du bord qui entoure la découpe (15) correspond au moins à un quart de son extension (26) dans la direction AP.

6. Prothèse intervertébrale selon l'une des revendications 1 à 5, **caractérisée en ce que** l'âme (20) de l'articulation est située essentiellement complètement à l'intérieur de la découpe (16).

7. Prothèse intervertébrale selon la revendication 6, **caractérisée en ce que** l'âme (20) de l'articulation porte un collet (25) qui empêche le contact direct entre les plaques de raccordement (1, 5) en cas de flexion.

8. Prothèse intervertébrale selon l'une des revendications 1 à 7, **caractérisée en ce que** la découpe (16) est rectangulaire et **en ce que** ses côtés s'étendent parallèlement ou perpendiculairement à la direction AP.

9. Prothèse intervertébrale selon la revendication 8, **caractérisée en ce que** la découpe (16) est carrée et **en ce qu'**elle dispose d'une âme d'articulation (b) délimitée en rectangle dont une paire de côtés (21) présente une longueur (26, 27) qui correspond essentiellement à celle de la découpe (16) et dont l'autre paire de côtés (22) présente une longueur plus courte.

10. Prothèse intervertébrale selon la revendication 8, **caractérisée en ce qu'**elle présente une âme d'articulation (a) dont les dimensions correspondent essentiellement à celles de la découpe (16).

11. Prothèse intervertébrale selon la revendication 8, **caractérisée en ce que** la découpe (16) est délimitée en carré et **en ce qu'**elle présente une âme (b) d'articulation délimitée en rectangle dont les côtés ont dans les deux directions une longueur plus courte que la découpe (16).
